# FASCICULE DE BREVET EUROPEEN

(11) **EP 2 552 542 B1**
(45) Date de publication et mention de la délivrance du brevet: **16.07.2014**
(21) Numéro de dépôt: 11718425.9
(22) Date de dépôt: 24.03.2011
(51) Int. Cl.: A61N 1/39, A61B 19/02, G08B 21/02, G08B 25/01, G08B 25/08, G06F 19/00, A62C 37/50, A61F 17/00

(54) **DISPOSITIF D'ACCES A UN MATERIEL D'ASSISTANCE ET DE SECOURS, ET UN SYSTEME METTANT EN OEUVRE DE TELS DISPOSITIFS.**
VORRICHTUNG FÜR DEN ZUGANG ZU EINER HILFS- UND NOTFALLSAUSRÜSTUNG SOWIE SYSTEM MIT DERARTIGEN VORRICHTUNGEN
DEVICE FOR ACCESS TO AN ITEM OF ASSISTANCE AND EMERGENCY HARDWARE, AND A SYSTEM IMPLEMENTING SUCH DEVICES.

(30) Priorité: 29.03.2010 FR 1052274
(43) Date de publication de la demande: 06.02.2013
(73) Titulaire: Atialis, 75009 Paris (FR)
(72) Inventeur: BROSSON, Hubert, F-75017 Paris (FR); DUVERDIER, Hubert, F-78400 Chatou (FR)
(74) Mandataire: Pontet Allano & Associes
(86) Numéro de dépôt international: PCT/FR2011/050630
(87) Numéro de publication internationale: WO 2011/124808

(56) Documents cités:
- DE-U1-202004 002 106
- US-A1- 2003 025 602
- US-A1- 2005 006 109
- US-A1- 2006 149 323
- US-A1- 2006 215 024
- US-A1- 2006 250 271

## Description

La présente invention concerne un dispositif d'accès à un matériel d'assistance et de secours, prévu pour être disposé à un emplacement donné. Elle concerne également un système mettant en oeuvre de tels dispositifs.

Le domaine de l'invention est le domaine de l'accessibilité à des appareils de secours et plus particulièrement des bornes d'accès à du matériel de secours mises à la disposition du public dans tout type de lieux publics.

Il existe actuellement quelques solutions mettant à la disposition du public des appareils de secours tels que des défibrillateurs. Les gestes de premiers secours sont essentiels, par exemple pour sauvegarder les fonctions vitales d'un sujet ou diminuer les dégâts causés par un événement tel qu'un incendie, dans l'attente de l'intervention du personnel de secours qualifié.

Les pouvoirs publics désirent améliorer l'accessibilité à des appareils de secours Grand Public pour améliorer le temps et la qualité d'intervention sur un sujet qui a besoin de tels matériels et/ou assistance secours en vue de diminuer le taux de mortalité et d'accident. Plus le temps d'intervention pour la délivrance de soins d'urgence est court, plus les chances de sauvegarder les fonctions vitales sont importantes.

Cependant, diminuer le temps d'intervention est directement corrélé à la présence et à la facilité d'accès du matériel de secours grand public. Par ailleurs, le matériel de premier secours doit être varié, et comprendre, par exemple, un nécessaire à stopper les brûlures, des couvertures anti feu, un extincteur, un défibrillateur, etc. Il est, en effet, impossible de prévoir quel type d'intervention d'urgence il sera nécessaire de porter, ni le lieu d'intervention.

Par ailleurs, l'accessibilité Grand Public à du matériel de secours varié, pose la question de la surveillance, de la maintenance et de la sécurité de ce matériel, souvent onéreux, face aux agressions et divers actes de vandalisme.

Les documents US 2006./215024 A1 et US 2006/250271 A1 décrivent, chacun, un dispositif d'accès à du matériel de premier secours comprenant plusieurs compartiments prévus, pour recevoir un matériel de premiers secours. L'ensemble des compartiments est accessible par une même et unique porte. On connait également le dispositif du document US 2006/149323 A1 qui comporte un unique compartiment pour recevoir un matériel de premier secours.

Il n'existe à ce jour aucun dispositif d'accès à du matériel de secours permettant de mettre à la disposition du public du matériel adapté tout en le préservant et en garantissant sa fonctionnalité.

Un but de la présente invention est de remédier à ces inconvénients.

Un autre but de l'invention est de proposer un dispositif d'accès à du matériel de premier secours varié tout en préservant ce matériel contre des actes malveillants.

Enfin, un autre but de l'invention est de proposer un meilleur accès à l'utilisation du matériel de premier secours.

L'invention permet d'atteindre ces buts par un dispositif d'accès à du matériel de secours, prévu pour être disposé à un emplacement donné, dit site de secours, comprenant:
- au moins deux compartiments, chacun d'eux étant prévu pour recevoir au moins un matériel de secours et agencé pour pouvoir être ouvert indépendamment,
- des moyens de communication avec un site distant, dit central, pour demander l'ouverture d'au moins un desdits compartiments, et caractérisé en ce qu'il comprend :
- pour chacun desdits compartiments, des moyens de verrouillage pouvant être commandés à distance et indépendamment de sorte que chaque compartiment puisse être ouvert indépendamment des autres.

Ainsi, le dispositif selon l'invention permet d'accéder, de manière sélective, à du matériel de secours se trouvant dans un compartiment.

Par ailleurs, l'accès au matériel de secours Grand Public est réalisé sur demande et après autorisation, et ce, de manière spécifique à chaque cas d'urgence. Chacun des compartiments pouvant être ouvert indépendamment des autres, il est ainsi possible de délivrer du matériel de secours de façon ciblée.

Enfin, le matériel de secours est stocké dans des compartiments qui sont, en tant normal, fermés et qui ne s'ouvriront que sur demande. Ainsi, le matériel de secours est protégé contre des actes de vandalisme.

Les moyens de verrouillage dont l'ouverture peut être commandée à distance peuvent comprendre au moins un électro-aimant, une gâche coulissante, une gâche crochetée, une crémaillère basculante.

Avantageusement, au moins un compartiment est prévu pour recevoir un nécessaire de défibrillation et au moins un autre compartiment est prévu pour recevoir des moyens de lutte incendie.

Selon une version particulièrement avantageuse du dispositif de l'invention, les moyens de communications peuvent en outre comprendre des moyens d'établissement et de réalisation d'une communication audio et/ou visuelle avec le site central suite à une demande d'ouverture, et plus particulièrement une communication en temps réel avec un opérateur situé dans le site distant.

Ainsi, les moyens de communications peuvent comprendre un ou plusieurs écrans permettant de voir l'opérateur en temps réel lors de l'échange, une ou plusieurs caméras permettant à l'utilisateur d'être vu en temps réel par l'opérateur ainsi qu'un ou plusieurs haut-parleurs et un ou plusieurs microphones permettant à l'utilisateur de parler avec l'opérateur en temps réel. Les moyens d'établissement de communication peuvent comprendre des moyens filaires ou des moyens sans fil au travers d'un réseau de télécommunication.

L'utilisateur est ainsi accompagné et aidé par un opérateur lors de l'accès au matériel de secours désiré. L'opérateur peut ainsi donner des indications en temps réel à l'utilisateur dans un moment d'urgence, ce qui rassure l'utilisateur et permet de le calmer lui permettant de mieux utiliser le matériel de secours.

Par ailleurs, l'opérateur peut décharger l'utilisateur de certains actes tels que l'appel aux services de secours professionnels, permettant ainsi à l'utilisateur de mieux se concentrer sur les gestes à prodiguer.

Avantageusement, le dispositif selon l'invention peut en outre comprendre des moyens de géo-localisation prévus pour signaler la position dudit dispositif au site central ou un autre site au travers d'un réseau de communications.

Par ailleurs, le dispositif selon l'invention peut en outre comprendre des moyens de connexion sans fil à un appareil se situant à proximité dudit dispositif. De tels moyens de connexion peuvent intégrer, par exemple, des liaisons dites Wi-fi, Bluetooth, et autres en vue, entre autres, de transférer à cet appareil des contenus de tous types, comme des informations relatives à l'utilisation d'appareils de secours.

De par cette spécificité, il est possible d'assurer la localisation par des contenus relatifs à des indications de direction visant à atteindre une adresse donnée. Ainsi, le dispositif selon l'invention permet aussi de porter assistance à des personnes qui se sont perdues.

Le dispositif selon l'invention peut en outre comprendre des moyens de diffusion de contenus audio et/ou visuels via des écrans ou des hauts parleurs.

Le dispositif peut comprendre des moyens de stockage de contenus au niveau du site de secours.

Les contenus peuvent être transmis depuis le site central ou un autre site distant au travers d'un réseau de communications filaire ou sans fil.

Avantageusement, au moins un compartiment du dispositif peut comprendre des moyens de vérification du fonctionnement d'un appareil lorsque ledit appareil est disposé dans ledit compartiment.

Selon un exemple de réalisation particulier et non limitatif, les défibrillateurs comprennent au moins un voyant permettant de signaler leur état de fonctionnement. Le compartiment peut alors comprendre un capteur optique ou autre, positionné de sorte à surveiller ce voyant et ainsi déterminer l'état de fonctionnement du défibrillateur en temps réel.

Par ailleurs, les moyens de vérification peuvent être couplés à un module de signalisation prévu pour remonter les signaux d'état de fonctionnement dudit appareil à un site de gestion distant.

Selon une première version le module de surveillance permet de signaler l'état de fonctionnement en temps réel.

Selon une autre version, le module de surveillance est adapté pour analyser les informations envoyées par les moyens de vérification et de signaler seulement un état de mal fonctionnement ou de dysfonctionnement.

Selon encore une autre version, le module de surveillance peut être interrogé à distance pour signaler l'état de fonctionnement d'appareils de secours.

Ainsi, le dispositif selon l'invention, permet de s'assurer que le matériel de secours se trouvant dans les compartiments fonctionne bien et pourra effectivement servir lors d'une urgence.

Les moyens de défibrillation peuvent comprendre un défibrillateur. Le défibrillateur peut être disposé dans une housse adaptée.

Les moyens de lutte incendie peuvent comprendre un extincteur et/ou une couverture anti feu ainsi que des produits pour arrêter ou soigner les brûlures.

Dans un mode de réalisation particulier, le dispositif selon l'invention peut se présenter sous la forme d'une borne disposée sur un emplacement public.

Selon les fonctionnalités mises en oeuvre, cette borne peut comporter, de manière non exhaustive :
- une base comportant les différents compartiments,
- un bouton d'appel d'urgence,
- au moins un haut-parleur et un microphone,
- une caméra,
- deux écrans/dalles LCD (anti vandalisme) disposés verticalement de chaque côté de la borne,
- au moins une antenne de connexion sans fil à un réseau de communication permettant une communication avec le site distant,
- au moins une antenne de connexion sans fil par Wifi ou Bluetooth à un appareil se trouvant à proximité, et
- des interfaces de communications électroniques et/ou informatiques.

Selon un autre aspect de l'invention, il est proposé un système d'accès à du matériel de secours comprenant :
- plusieurs dispositifs d'accès à du matériel de secours selon l'invention, chacun étant disposé sur un site, dit site de secours,
- des moyens de communication disposés au niveau d'un site, dit central, distant desdits sites de secours, lesdits moyens de communication étant en liaison avec lesdits dispositifs de secours au travers d'au moins un réseau de communication.

Le site central comprend en outre des moyens pour commander à distance l'ouverture d'un compartiment d'un dispositif de secours suite à une demande.

D'autres avantages et caractéristiques apparaîtront à l'examen de la description détaillée d'un mode de réalisation nullement limitatif, et des dessins annexés sur lesquels
- la figure 1 est une représentation schématique sous forme de blocs d'un dispositif selon l'invention ;
- la figure 2 est une représentation schématique d'un dispositif selon l'invention se présentant sous la forme d'une borne ; et
- la figure 3 est une représentation schématique d'un système selon l'invention mettant en oeuvre plusieurs dispositifs selon l'invention.

Sur les figures les éléments communs à plusieurs figures conservent la même référence.

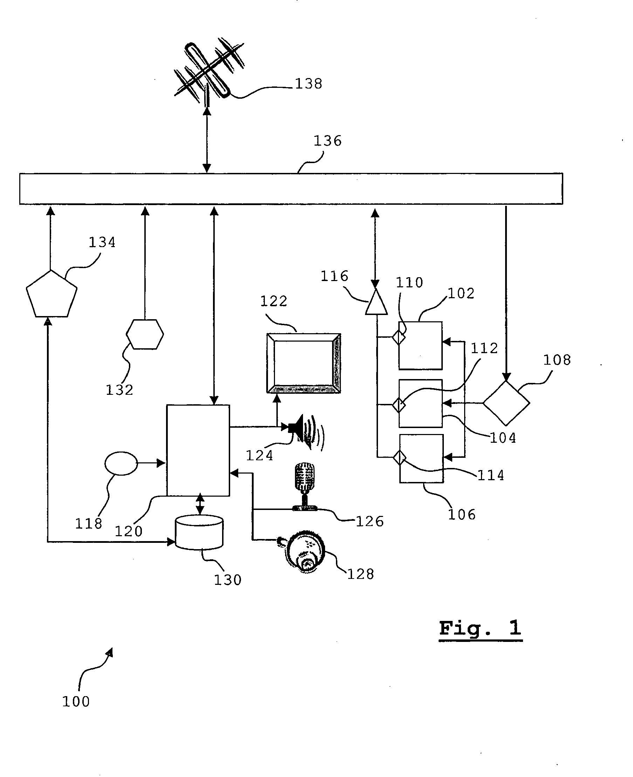

La figure 1 est une représentation schématique sous forme de blocs d'un exemple d'un dispositif 100 d'accès à du matériel de premiers secours selon l'invention.

Le dispositif 100 comporte trois compartiments 102, 104 et 106, prévus pour contenir du matériel de secours tels qu'un défibrillateur, un extincteur, etc.

Chaque compartiment est verrouillé. L'ouverture de chaque compartiment peut être réalisée, de manière sélective et individuelle, à distance grâce à un module 108 de gestion relié aux compartiments 102-106. Le module de gestion est relié à des moyens de verrouillage (non représenté) de chacun des compartiments 102-106 qui peuvent être commandés à distance. Ces moyens de verrouillage peuvent, par exemple, comprendre des moyens de verrouillage électriques mettant en oeuvre des vérins ou des aimants actionnés électriquement. Ces types de moyens de verrouillage sont connus de l'homme du métier.

Chaque compartiment 102-106 comprend un capteur, respectivement 110-114, disposé dans le compartiment 102-106 pour capter des signaux de fonctionnement d'un appareil disposé dans le compartiment 102-106. Les capteurs 110-114 sont reliés à un module de signalisation 116, qui permet à tout moment de signaler l'état de fonctionnement de chacun des appareils disposés dans chacun des compartiments, de manière individuelle.

Le dispositif 100 comporte en outre un bouton d'appel 118 relié à un module de communication 120 qui permet de générer un appel vers un site distant.

Le dispositif 100 comprend en outre au moins un écran d'affichage 122, au moins un haut-parleur 124, au moins un microphone 126 et au moins une caméra 128 qui sont gérés par le module de communication pour établir et réaliser une communication audiovisuelle en temps réel avec un opérateur situé sur un site distant, qui sera appelé site central dans la suite de la description.

L'écran d'affichage 122 et le haut-parleur 124 sont en outre agencés pour diffuser des contenus qui peuvent être soit diffusés en temps réel, soit préenregistrés dans des moyens de mémorisation 130.

Le dispositif 100 comprend en outre un module de géo-localisation 132 permettant de déterminer et d'émettre des données relatives à la localisation du dispositif 100.

Par ailleurs, le dispositif 100 comprend en outre un module 134 de connexion à des appareils se trouvant à proximité du dispositif 100 par une connexion Wifi, Bluetooth ou autres pour le téléchargement de contenus enregistrés dans les moyens de mémorisation 130. Un tel module 134 peut également servir de point de connexion à un réseau de type internet pour les appareils en question afin d'accéder à des données d'itinéraires ou autres.

Enfin, le dispositif 100 comprend une interface 136 de gestion des communications et une antenne 138 pour la réception et l'émission des signaux échangés avec un site central au travers d'un réseau communications par ondes ou avec un appareil se trouvant à proximité. Ainsi, dans l'exemple représenté sur la figure 1, l'interface de gestion 136 est reliée à l'antenne commune 138, au module de communication 120, au module de géo-localisation 130, au module 134 de connexion de proximité, au module de signalisation 116 et au module 108 de gestion de l'ouverture des compartiments 102-106. L'interface de gestion 136 permet d'aiguiller les différents signaux reçus par l'antenne 138 vers les différents modules et de transmettre les signaux émis par les différents modules vers l'antenne 138.

Nous allons maintenant décrire le fonctionnement du dispositif 100.

Lorsqu'un cas d'urgence se produit à proximité du dispositif 100, un utilisateur actionne le bouton d'appel 118. Le signal d'appel est géré par le module de communication 120, puis l'interface de gestion 136 et émis par l'antenne 138 vers le site central. Une communication audiovisuelle est mise en place entre le dispositif 100 et le site central par l'utilisation de l'antenne 138, de l'interface 136 et du module de communication 120. Grâce au microphone 126, l'utilisateur peut alors parler à un opérateur dont l'image s'affiche sur l'écran d'affichage 122. L'utilisateur entend également l'opérateur grâce au haut-parleur 124. L'opérateur peut également voir l'utilisateur grâce à la caméra 128.

L'utilisateur indique alors à l'opérateur l'objet de son appel et le matériel dont il a besoin pour les gestes de premiers secours.

L'opérateur émet alors un signal d'ouverture qui comprend l'indication du compartiment concerné. Ce signal est reçu par l'antenne 138 puis par l'interface de gestion 136. L'interface de gestion 136 transmet le signal au module 108 de gestion de l'ouverture de compartiments 102-106. Une fois reçu, le module 108 analyse le signal d'ouverture, détermine le compartiment à ouvrir et déclenche l'ouverture du compartiment.

L'utilisateur a alors accès au matériel se trouvant dans le compartiment et peut l'utiliser. Pendant ce temps, l'opérateur peut rassurer l'utilisateur, le calmer et l'assister en alertant les secours à sa place. L'utilisateur peut ainsi se concentrer entièrement sur les gestes de premiers secours.

Par ailleurs, pour déterminer la localisation du dispositif, l'opérateur émet une requête pour la réception des données de localisation. Cette requête est reçue par l'antenne 138, puis l'interface de gestion 136. L'interface de gestion 136, transmet la requête au module de géo-localisation 132. En réponse à cette requête, le module de géo-localisation 132 émet des données de localisation, qui sont transmises par l'interface de gestion 136, puis l'antenne 138, vers le site central.

Lorsque le dispositif 100 n'est pas utilisé des contenus mémorisés sur des moyens de mémorisation sont diffusés sur l'écran 122 et le haut-parleur 124.

Par ailleurs, les contenus mémorisés peuvent également être transférés à des appareils se trouvant à proximité du dispositif 100 grâce à une connexion Bluetooth ou Wifi, entre autres, assurée par le module 134 de connexion de proximité.

Ces contenus peuvent concerner des données d'itinéraires, des données de formation à l'utilisation du matériel de secours Grand Public, des contenus audiovisuels ou autres.

De plus, chaque compartiment 102-106 comprend un capteur 110-114 agencé pour capter des signaux de fonctionnement d'un appareil disposé dans ce compartiment 102-106, par exemple un défibrillateur.

Les signaux captés par ces capteurs 110-114 sont transférés à un module de signalisation 116, qui analyse ces signaux et permet de déterminer l'état de fonctionnement des appareils disposés dans les compartiments 102-106. Le module 116 transmet, au site central, à intervalle régulier, des données relatives à l'état de fonctionnement des appareils se trouvant dans les compartiments 102-104.

Chaque compartiment 102-106 est étanche et est réalisé en matériau résistant à des actes de vandalisme. Chaque compartiment peut être réalisé en acier ou en inox striés, prétraitée antirouille pour l'acier. Chaque compartiment peut comporter un ou plusieurs joints étanches.

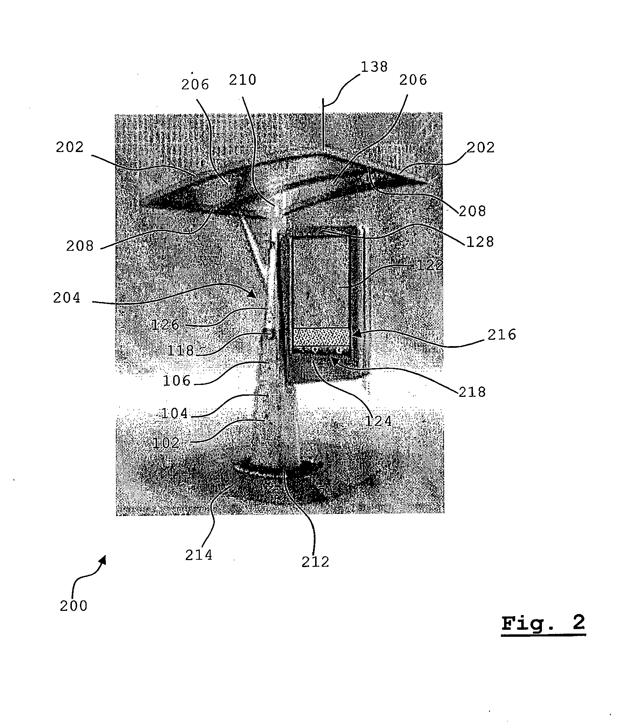

La figure 2 est une représentation schématique d'un dispositif selon l'invention se présentant sous la forme d'une borne 200.

Cette borne 200 est équipée d'un écran LCD 122 de type 46 pouces (soit 105cm x 60cm) disposé verticalement sur le côté de la borne. Le bas de l'écran 122 est disposé à une hauteur de 1m10 depuis le sol. De plus, le bouton d'appel 118 est disposé à une hauteur de 1m05. La borne 200 est protégée des intempéries par un bouclier 202 disposé au niveau de sa partie supérieure et supporté par un mat porteur 204. Le bouclier supérieur 202 se présente sous la forme d'un toit intégrant un ou plusieurs panneaux photovoltaïques 206 permettant d'alimenter une partie de la borne en électricité.

La borne comprend en outre des leds 208 disposés sur le toit 202 et permettant d'éclairer l'emplacement de la borne 200 la nuit. La borne comporte aussi un gyrophare 210.

La borne comporte en sa partie inférieure un socle 212 dit « platine » permettant de fixer la borne 200 au sol. Un revêtement de surface 214 antidérapante est disposé autour de la borne 200.

Par ailleurs, l'écran 122 comporte une zone tactile 216 et des boutons de commande 218 agencés pour qu'un utilisateur puisse interagir avec la borne 200 et/ou l'écran 122.

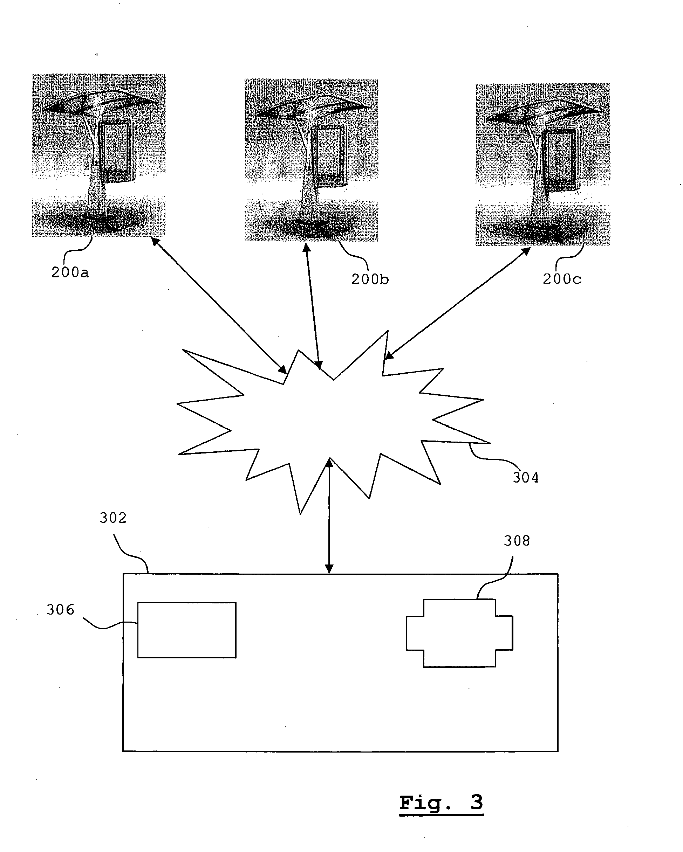

La figure 3 est une représentation d'un système 300 selon l'invention.

Le système 300 comprend un module central 302 localisé au niveau d'un site central. Le système comprend en outre, de manière non limitative, trois bornes 200a-200c d'accès à du matériel de secours grand public disposés sur trois sites de secours. Le module central 302 est en liaison avec chacune des bornes 200a-200c de secours au travers d'un réseau de communications sans fil 304.

Le module central 302 comprend des moyens 306 de communications audio-visuels permettant de réaliser des communications audiovisuels, en temps réel, entre un utilisateur situé sur un site de secours et un ou plusieurs opérateurs situé sur le site central. Le module central comprend en outre des moyens 308 d'émission d'une requête d'ouverture d'un compartiment d'une borne 200a-200c.

Bien entendu, l'invention n'est pas limitée aux exemples détaillés ci-dessus.

## Revendications

1. Dispositif (100, 200, 200a-200c) d'accès à du matériel de premier secours, prévu pour être disposé à un emplacement donné, dit site de secours, comprenant:
- au moins deux compartiments (102-106), chacun des compartiments (102-106) étant prévu pour recevoir au moins un matériel de premiers secours et agencé pour pouvoir être ouvert indépendamment,
- des moyens de communication (120-128) avec un site distant, dit central, pour demander l'ouverture d'au moins un desdits compartiments (102-106) auprès d'un site distant, et **caractérisé en ce qu'**il comprend:
- pour chacun desdits compartiments (102-106), des moyens de verrouillage pouvant être commandés à distance et indépendamment de sorte que chaque compartiment puisse être ouvert indépendamment des autres compartiments.

2. Dispositif (100) selon la revendication 1, **caractérisé en ce que** les moyens de communications (120-128) comprennent en outre des moyens d'établissement et de réalisation d'une communication audio et/ou visuelle avec le site distant suite à une demande d'ouverture.

3. Dispositif (100) selon l'une quelconque des revendications précédentes, **caractérisé en ce qu'**il comprend en outre des moyens de géo-localisation (132) prévus pour signaler la position dudit dispositif (100) au site central ou un autre site au travers d'un réseau de communications.

4. Dispositif selon l'une quelconque des revendications, **caractérisé en ce qu'**il comprend en outre des moyens (134) de connexion sans fil à un appareil se situant à proximité dudit dispositif (100).

5. Dispositif selon l'une quelconque des revendications, **caractérisé en ce qu'**il comprend en outre des moyens (120, 122, 124, 130) de diffusion de contenus audio et/ou visuels.

6. Dispositif selon l'une quelconque des revendications précédentes, **caractérisé en ce qu'**au moins un compartiment (102-106) comprend des moyens (110-114) de vérification du fonctionnement d'un appareil lorsque ledit appareil est disposé dans ledit compartiment (102-106).

7. Dispositif selon la revendication 6, **caractérisé en ce que** les moyens de vérification (110-114) sont couplés à un module de signalisation (116) prévu pour signaler l'état de fonctionnement dudit appareil à un site de gestion distant.

8. Dispositif selon l'une quelconque des revendications précédentes, **caractérisé en ce que** le matériel de premier secours comprend un défibrillateur disposé dans un premier compartiment (102-106).

9. Dispositif selon l'une quelconque des revendications précédentes, **caractérisé en ce que** le matériel de premier secours comprend un extincteur et/ou une couverture anti feu disposé(s) dans un deuxième compartiment (102-106).

10. Dispositif (100) selon l'une quelconque des revendications précédentes, **caractérisé en ce qu'**il se présente sous la forme d'une borne (200) disposée sur en emplacement public.

11. Système (300) d'accès à du matériel de premier secours, ledit système comprenant :
- plusieurs dispositifs (200a-200c) d'accès à du matériel de premier secours selon l'une quelconque des revendications précédentes, chacun étant disposé sur un site, dit de secours,
- des moyens de communication (306) disposés au niveau d'un site, dit central, distant desdits sites de secours, lesdits moyens de communication (306) étant en liaison avec lesdits dispositifs (200a-200c) de secours au travers d'au moins un réseau de communication (304), et
- des moyens (308) pour commander à distance l'ouverture d'un emplacement d'un dispositif de secours suite à une demande.

## Patentansprüche

1. Vorrichtung (100, 200, 200a-200c) für den Zugang zu Erste-Hilfe-Ausrüstung, die dazu vorgesehen ist, an einem gegebenen Standort, sogenannten Rettungsort, angeordnet zu werden, umfassend:
- wenigstens zwei Fächer (102-106), wobei ein jedes der Fächer (102-106) dazu vorgesehen ist, wenigstens eine Erste-Hilfe-Ausrüstung aufzunehmen, und dazu eingerichtet ist, unabhängig geöffnet zu werden,
- Mittel zur Kommunikation (120-128) mit einem entfernten, sogenannten zentralen Ort, um das Öffnen von wenigstens einem der Fächer (102-106) bei einem entfernten Ort anzufordern, und
**dadurch gekennzeichnet, dass** sie umfasst:
- für ein jedes der Fächer (102-106) Verriegelungsmittel, die fern- und unabhängig gesteuert werden können, so dass jedes Fach unabhängig von den anderen Fächern geöffnet werden kann.

2. Vorrichtung (100) nach Anspruch 1, **dadurch gekennzeichnet, dass** die Kommunikationsmittel (120-128) ferner Mittel zum Aufbauen und Führen einer Hör- und/oder Sichtverbindung bzw. -kommunikation mit dem entfernten Ort infolge einer Öffnungsanfrage umfassen.

3. Vorrichtung (100) nach einem der vorhergehenden Ansprüche, **dadurch gekennzeichnet, dass** sie ferner Geolokalisierungsmittel (132) umfasst, die dazu vorgesehen sind, die Position der Vorrichtung (100) dem zentralen Ort oder einem anderen Ort über ein Kommunikationsnetz zu melden.

4. Vorrichtung nach einem der vorhergehenden Ansprüche, **dadurch gekennzeichnet, dass** sie ferner Mittel (134) zur drahtlosen Verbindung mit einem Gerät, das sich in der Nähe der Vorrichtung (100) befindet, umfasst.

5. Vorrichtung nach einem der vorhergehenden Ansprüche, **dadurch gekennzeichnet, dass** sie ferner Mittel (120, 122, 124, 130) zur Übertragung von Audio- und/oder visuellen Inhalten umfasst.

6. Vorrichtung nach einem der vorhergehenden Ansprüche, **dadurch gekennzeichnet, dass** wenigstens ein Fach (102-106) Mittel (110-114) zur Überprüfung des Betriebs eines Gerätes, wenn das Gerät in dem Fach (102, 106) angeordnet ist, umfasst.

7. Vorrichtung nach Anspruch 6, **dadurch gekennzeichnet, dass** die Überprüfungsmittel (110-114) mit einem Meldemodul (116) gekoppelt sind, das dazu vorgesehen ist, den Betriebszustand des Gerätes einem entfernten Verwaltungsort zu melden.

8. Vorrichtung nach einem der vorhergehenden Ansprüche, **dadurch gekennzeichnet, dass** die Erste-Hilfe-Ausrüstung einen Defibrillator, welcher in einem ersten Fach (102-106) angeordnet ist, umfasst.

9. Vorrichtung nach einem der vorhergehenden Ansprüche, **dadurch gekennzeichnet, dass** die Erste-Hilfe-Ausrüstung einen Feuerlöscher und/oder eine Löschdecke, die in einem zweiten Fach (102-106) angeordnet ist (sind), umfasst.

10. Vorrichtung (100) nach einem der vorhergehenden Ansprüche, **dadurch gekennzeichnet, dass** sie in Form einer Säule (200), die an einem öffentlichen Standort angeordnet ist, vorliegt.

11. System (300) für den Zugang zu Erste-Hilfe-Ausrüstung, wobei das System umfasst:
- mehrere Vorrichtungen (200a-200c) für den Zugang zu Erste-Hilfe-Ausrüstung nach einem der vorhergehenden Ansprüche, wobei eine jede an einem sogenannten Rettungsort angeordnet ist,
- Kommunikationsmittel (306), die im Bereich eines von den Rettungsorten entfernten, sogenannten zentralen Ortes angeordnet sind, wobei die Kommunikationsmittel (306) über wenigstens ein Kommunikationsnetz (304) mit den Rettungsvorrichtungen (200a-200c) in Verbdingung stehen, und
- Mittel (308), um infolge einer Anfrage das Öffnen eines Standortes einer Rettungsvorrichtung fernzusteuern.

## Claims

1. A device (100, 200, 200a-200c) for access to first emergency response equipment, provided to be placed at a given location, called emergency response site, comprising:
- at least two compartments (102-106), each of the compartments (102-106) being provided for receiving at least one item of first emergency response equipment and arranged to be able to be opened independently,
- means of communication (120-128) with a remote site, called central site, for requesting the opening of at least one of said compartments (102-106) from said central site,
and **characterized in that** it comprises:
- for each of said compartments (102-106), locking means being able to be remotely and independently controlled so that each compartment can be opened independently of the other compartments.

2. The device (100) according to claim 1, **characterized in that** the communication means (120-128) include moreover means for establishing and carrying out an audio and/or visual communication with the remote site following a request for opening.

3. The device (100) according to any one of the previous claims, **characterized in that** it moreover comprises geolocation means (132) provided for signalling the position of said device (100) to the central site or another site via a communication network.

4. The device according to any one of the previous claims, **characterized in that** it comprises moreover means (134) for wireless connection to an appliance situated in proximity to said device (100).

5. The device according to any one of the previous claims, **characterized in that** it comprises moreover means (120, 122, 124, 130) for broadcasting audio and/or visual content.

6. The device according to any one of the previous claims, **characterized in that** at least one compartment (102-106) comprises means (110-114) for verifying the operational state of an apparatus when said apparatus is arranged in said compartment (102-106).

7. The device according to claim 6, **characterized in that** the verification means (110-114) are coupled to a signalling module (116) provided for signalling the operational state of said apparatus to a remote management site.

8. The device according to any one of the previous claims, **characterized in that** the first emergency response equipment item comprises a defibrillator arranged in a first compartment (102-106).

9. The device according to any one of the previous claims, **characterized in that** the first emergency response equipment item comprises a extinguisher and/or a fire blanket arranged(s) in a second compartment (102-106).

10. The device (100) according to any one of the previous claims, **characterized in that** it is presented in the form of a terminal (200) arranged in a public place.

11. A system (300) for access to first emergency response equipment items, said system comprising:
- several devices (200a-200c) for access to first emergency response equipment according to any one of the previous claims, each being arranged on a site, called emergency response site,
- communication means (306) arranged at a site, called central site, remote from said emergency response sites, said communication means (306) being linked with said devices (200a-200c) via at least one communication network (304),
- means (308) for remotely controlling the opening of a compartment of an emergency device following a request.
